Europäisches Patentamt

European Patent Office

Office européen des brevets

(19)

(11) Publication number: **0 254 335 B1**

(12) **EUROPEAN PATENT SPECIFICATION**

(45) Date of publication of patent specification: **08.01.92**     (51) Int. Cl.⁵: **B01J 27/128**, B01J 23/89

(21) Application number: **87201129.1**

(22) Date of filing: **15.06.87**

(54) **Process for preparation of a hydrogenation and/or dehydrogenation catalyst.**

(30) Priority: **11.07.86 SE 8603088**

(43) Date of publication of application:
**27.01.88 Bulletin 88/04**

(45) Publication of the grant of the patent:
**08.01.92 Bulletin 92/02**

(84) Designated Contracting States:
**AT BE CH DE ES FR GB GR IT LI LU NL SE**

(56) References cited:
**EP-A- 0 055 512**
**EP-A- 0 146 508**
**EP-A- 0 162 263**
**US-A- 4 268 699**

(73) Proprietor: **BEROL KEMI AB**
**Box 851**
**S-444 01 Stenungsund 1(SE)**

(72) Inventor: **Köll, Juhan**
**Gullvivev 18**
**S-444 00 Stenungsund(SE)**

(74) Representative: **Andersson, Rolf**
**BEROL KEMI AB Box 851**
**S-444 01 Stenungsund(SE)**

EP 0 254 335 B1

## Description

The present invention relates to a method for the preparation of a ruthenium promoted nickel and/or cobalt hydrogenation and/or dehydrogenation catalyst on a porous metal oxide support. Halogen is incorporated in the catalyst by adding a halide compound at any stage of the process in form of a non-ruthenium halide.

European patent application No. 146 508 describes a ruthenium promoted nickel or/and cobalt hydrogenation and/or dehydrogenation catalyst on a porous metal oxide support.

The catalyst is claimed as being obtainable by

a. impregnating the support coated with nickel and/or cobalt present as metals or oxides with a solution of a ruthenium halide compound, and

b. drying the catalyst intermediate, reducing the ruthenium halide compound at elevated temperature in a stream of hydrogen gas to ruthenium metal, and if necessary, finally reducing nickel and/or cobalt oxides in hydrogen gas to finely divided nickel and/or cobalt metal.

Said application points out that it cannot be stated with certainty, whether the advantageous results obtained through the use of said catalyst in an amination process, which involves one dehydrogenation step and one hydrogenation step, are related to the way in which cobalt and/or nickel and ruthenium are deposited on the support, or whether the metals and the support have undergone chemical reactions to give new physical and chemical characteristics to the catalyst. The patent application further points out that catalysts prepared in similar way, but using ruthenium compounds others than halides, give an inferior catalytic performance in the amination process.

It has now been found that the same catalyst as described in said patent application can be obtained by introducing a halide compound into the catalyst at any stage of the process. That means that the ruthenium compound used not necessarily needs to be a ruthenium halide. The advantageous properties of the catalyst seem to be related to the presence of the halide in it.

The present invention relates to a process for preparation of a ruthenium-promoted, halogen-containing, nickel and/or cobalt catalyst, containing from 4-40% by weight, based on the total weight of said catalyst, of at least one metal selected from nickel and cobalt; from 0.1-5% by weight, based on the total weight of said catalyst, of ruthenium; and a porous metal oxide support comprised of at least 50% by weight of activated alumina and/or silica, in which process the support is impregnated in one or more steps with a nickel compound and/or a cobalt compound and a ruthenium compound, and the nickel and/or the cobalt compounds and the ruthenium compound being reduced into finely divided nickel and/or cobalt and ruthenium metals, characterized in that a halide is introduced into the catalyst by adding a halide compound in any other form than a ruthenium halide compound at any stage of the process.

Thus, the treatment with halide compound may be performed at any step in the catalyst preparation procedure, such as

introducing the halide compound prior to the addition of the nickel or/and cobalt compound to the support,

impregnating the support with a mixture of nickel and/or cobalt compound and halide compound,

decomposing the supported nickel and/or cobalt compound into metal or oxide in an atmosphere containing suitable halide compound,

treating the catalyst intermediate comprising supported nickel and/or cobalt metal or oxide with the halide compound,

adding the halide compound to the ruthenium compound solution used for impregnation of the catalyst intermediate,

treating the catalyst intermediate containing supported nickel and/or cobalt metal or oxide and ruthenium metal with the halide compound before the final reduction step,

adding a volatile halide compound to hydrogen gas in the reduction step,

adding the halide compound to the impregnation solution containing nickel and/or cobalt compound and ruthenium compound in a combined impregnation step,

According to one preferred embodiment of the process the catalyst may be prepared by

first preparing by any conventional method a catalyst intermediate consisting of nickel and/or cobalt as finely divided metals or oxides on the porous metal oxide support,

then treating the thus prepared catalyst intermediate with a halide compound,

thereafter impregnating the halide treated catalyst intermediate with a soluble ruthenium compound, and

finally reducing the ruthenium compound, and nickel and/or cobalt oxides (if present) into corresponding finely divided metals.

Another preferred embodiment is to impregnate the support with nickel and/or cobalt compound, ruthenium compound, and a halide compound in one single solution. Subsequent calcination and reduction

2

will transfer nickel and/or cobalt, and ruthenium compounds simultaneously into finely distributed metals.

Primarily preferred halide compounds are hydrogen halides, such as hydrofluoric acid, hydrochloric acid, hydrobromic acid, and hydroiodic acid.

Other preferred halide compounds include halide salts of weak bases, which provide hydrogen halides at elevated temperatures. Examples of such compounds are ammonium fluoride, ammonium acid fluoride, ammonium chloride, ammonium bromide, and ammonium iodide.

Further suitable halide compounds are neutral or acid salts of hydrohalide acids that form hydrogen halides when exposed to acidic gases or liquids present or liberated during the catalyst preparation. Examples of such compounds are alkali metal halides, and alkaline earth metal halides.

Suitable halide compounds also comprise organic halogen compounds which can decompose into hydrogen halides. Examples of this group are organic acid chlorides, chlorinated hydrocarbons, and their derivatives, such as acetyl chlorides, tertiary butyl chloride, and chloroacetic acids.

Finally, inorganic compounds that will decompose into acidic halides may be used. Examples of such compounds are sulphuryl chloride and thionyl chloride.

The halide compound can be used in gas phase, as a solution in water or other suitable solvent, dissolved in solutions used in catalyst preparation, or as a liquid itself.

The choice of the halide compound type and amount, the treatment temperature and duration, and the way of treatment depends on the support material and shape, on available catalyst preparation equipment, on ruthenium compound used, and on availability of different halide compounds. The optimal parameters cannot be given in general, but must be determined individually for each combination of variables. This optimum may vary for different applications for which the catalyst is to be used.

However, numerous tests have shown, that halide amounts (calculated as weight percent of elemental halogen in final catalyst) between about 0.1 and about 5 percent are generally preferred. Most preferred amounts are from 0.25 to 2.5 percent. The addition of the halide compound and introduction of the halide ion in the catalyst should preferably be performed in such a way that the above mentioned halide amounts are obtained in the catalyst.

As ruthenium compound such ruthenium compounds can be used which are soluble in water, organic solvents or volatile acids, and transferable into metallic ruthenium e.g. by reducing the ruthenium compound in hydrogen gas at an elevated temperature. The temperature for converting ruthenium compound into metallic ruthenium has to be chosen to suit the particular ruthenium compound used, but normally those ruthenium compounds are chosen which are convertible to metallic form at a temperature between 100 and 400°C. Most preferred non-halide ruthenium compounds, considering availability and price, are ruthenium and ruthenium nitrosyl nitrate.

According to the invention any conventional metal oxide support containing at least 50% by weight of alumina and/or silica may be used. The metal oxide support materials, which have been found to produce the most active and selective amination catalysts are those containing more than 95% activated alumina. Examples of such supports are those consisting of alumina/silica, alumina/titania, alumina/magnesia, alumina/zirconia, and other combinations. The inner surface area of the catalyst support is not critical and may vary from 10 to 1000, preferably 20 to 400 square meters per gram support, but the area is suitably adapted to the metal amount in order to achieve a mainly monomolecular layer of catalyst metals on the support. A number of such support materials are well-known in the art and are also commercially available.

The chemical structure of the support materials largely influence the catalyst properties. As an example carbon supported ruthenium promoted nickel and/or cobalt catalysts show no selectivity on primary amines but on contrary promote formation of secondary and tertiary amines. Other supports of mainly acid nature may even give better activity with ruthenium promotion than without, but the catalysts made from these supports are less selective than those built on metal oxides.

The support material used in the invention may be co-precipitated with nickel and/or cobalt salts, or these metals can be transferred to the support by impregnation with solutions of metal salts. Various organic and inorganic nickel and cobalt salts may be used for coprecipitation or impregnation. Examples of suitable salts are nickel nitrate, nickel acetate, nickel formate, and nickel acetonyl acetate as well as corresponding cobalt salts. Nickel chloride and/or cobalt chloride may be used, but these salts are not decomposed by heating in air. Instead they can be transferred into metal by heating in hydrogen gas. Another method of depositing metals on the support is using nickel or cobalt carbonyl gas and decomposing it on the surface of the support to extremely finely divided metal. In accordance with the invention nickel and cobalt may be used alone, in admixture with each other, or one of them may be put on top of the other one. Which metal and which application method that will give the best result in each single amination process cannot be predicted but must be determined experimentally. As long as recognized principles of hydrogenation catalyst manufacture are used, the particular method of impregnating or coating the nickel or cobalt metal onto the support material has not been found to have any significant effect on the activity or selectivity of the final catalyst.

The amount of nickel and/or cobalt to be used depends on the composition and physical characteristics such as surface area and pore distribution of the catalyst support. In most cases the most active catalysts have been found to be those in which the contents of nickel and/or cobalt are between 5 and 20 percent of total catalyst weight and of ruthenium between 0.2 and 3 percent of total catalyst weight on a support having 50-100 square meters inner area per gram. The amount of the nickel and/or cobalt metal on the support has mainly an effect on the activity of the catalyst and less on the selectivity.

The support material impregnated with the desired amount of nickel and/or cobalt salt may be dried and then calcinated to decompose the salts into metal oxides. This can be accomplished by heating the catalyst first gently and, if desired, under reduced pressure to evaporate the impregnating solvent, then in a stream of air raising the temperature to 300-600° C depending on the decomposition temperature of the salt used and keeping that temperature until the salt is completely transferred into oxides. It is essential for the result that minor amounts of salts used, especially of nitrates, do not remain undecomposed after the calcination. It is also possible to transfer the oxides formed into metals by reacting the catalyst intermediate with hydrogen gas at elevated temperature.

In order to transform nickel and/or cobalt oxides into finely divided metal, the nickel and/or cobalt oxides may be reduced in a stream of hydrogen at elevated temperature. Preferably the reduction is carried out between 300 and 600° C for such a period of time that the desired degree of reduction is reached. Usually a high degree of reduction is preferred, but because of sintering of the support material and nickel and cobalt powder at prolonged heating, resulting in decreased surface area, a lower degree of reduction is sometimes tolerated. In case cobalt and/or nickel is present in metallic form at the impregnation with the ruthenium compound, only reduction of ruthenium is necessary.

The activated catalyst is best handled in the absence of air in order to prevent the reoxidation of nickel or cobalt. The catalyst may also be stabilized by gentle oxidation, carbon dioxide treatment, or other conventional techniques for stabilizing pyrophoric catalysts, and may then be handled in air prior to its utilization.

The catalysts procuded in accordance with the present process exhibit excellent hydrogenation and/or dehydrogenation properties. They can advantageously be used in amination reactions, e.g. by reacting an alkylene oxide, an hydroxyl containing compound, an aldehyde or a ketone with ammonia, a primary amine or a secondary amine.

The invention is further illustrated by the following examples using the amination of monoethanolamine to ethyleneamines as a measure of the catalyst efficiency. The abbreviations used in examples and the table are.

EDA = Ethylenediamine
MEA = Monoethanolamine
PIP = Piperazine
DETA = Diethylenetriamine
AEP = Aminoethyl piperazine
AEEA = Aminoethyl piperazine
HEP = Hydroxyethyl piperazine

The conversion is defined as the amount MEA consumed in the reaction in percent of MEA originally charged.

Example 1 Step A. Nickel impregnation

A saturated aqueous nickel nitrate solution containing one weight part of nickel, calculated as metal, was added to 9 weight parts of a catalyst support, consisting of 95 percent gamma alumina. The support was in form of tablets with length and diameter of 3 mm and a total surface area of about 100 square meters per gram of support.

Excess of the water was evaporated in vacuum at about 75° C, the tablets were dried, and the nickel nitrate was decomposed by heating in air at 500° C to nickel oxide. This alumina support, coated with finely distributed nickel oxide was used as catalyst intermediate also in examples 2 to 6, and in comparative examples A and B.

Step B. Acid treatment

To the catalyst intermediate was added twice its weight of 18 percent aqueous hydrochloric acid solution. After 30 minutes at room temperature the excess liquid was removed, the tablets were dried at 110°C and cooled.

Step C. Ruthenium impregnation

The tablets were impregnated using a two percent aqueous solution of ruthenium nitrosyl nitrate, containing 0.5 percent of ruthenium, calculated as metal and on the weight of alumina support used. The tablets were then dried at 110°C in air.

Step D. Reduction with hydrogen gas.

The tablets were heated in a stream of hydrogen gas, first for one hour at about 180-200°C, then for four hours at 400°C in order to reduce the main part of nickel and ruthenium salts in the tablets to metals in finely dispersed form.

Step E. Catalyst testing.

A 300 ml autoclave, equipped with a stirrer and temperature control, was flushed with nitrogen gas. Eight grams of the catalyst to be tested, 25 grams of MEA, 3.5 grams of water, and 65 grams of liquid ammonia were charged into the autoclave. The autoclave was closed, and hydrogen gas was introduced to a pressure of 5.5 MPa. The contents of the autoclave was heated to 200°C and kept at this temperature with continuous stirring until the completion of the test.

Samples were withdrawn from the autoclave during the reaction and analyzed using Gas Liquid Chromatography. The conversion of MEA was calculated as well as weight percents of products formed in the reaction. From these figures the ratios of primary, secondary, and tertiary amino groups to total amino groups in products formed, given as mole percent, were calculated and reported.

The results obtained are reported in the table.

Comparative example A.

A catalyst was prepared as described in Example 1, but the hydrochloric acid treatment (Example 1, Step B) was omitted. The results obtained are given in the table.

Comparative example B.

(Catalyst according to EP. Appl. No. 146 508)

A catalyst was prepared as described in Example 1, but hydrochloric acid treatment was omitted and ruthenium nitrosyl nitrate was replaced by ruthenium chloride hydrate containing the same amount of ruthenium metal as given in Example 1, Step C. The results obtained are given in the table.

Examples 2 - 4

Three catalysts were prepared as described in Example 1, but hydrochloric acid was replaced by the same molar amount of hydrofluoric acid (Example 2), hydrobromic acid (Example 3), or hydroiodic acid (Example 4). The results obtained are reported in the table.

Example 5

A catalyst was prepared as described in Example 1, but 18 percent aqueous hydrochloric acid solution was replaced by the same volume of 25 percent aqueous ammonium chloride solution. The results obtained are given in the table.

Example 6

A catalyst was prepared as described in Example 1, but 18 percent aqueous hydrochloric acid solution was replaced by the same volume of 10 percent aqueous sodium chloride solution. The results obtained are given in the table.

Example 7-11

Catalysts were prepared according to Example 1, but the hydrochloric acid treatment (Example 1, Step B) was performed at different stages of the catalyst preparation.

Example 7

The same alumina support as used in Example 1, was first treated with hydrochloric acid in the same way as described in Example 1, Step B. Then this support was impregnated first with nickel as described in Example 1, Step A, then with ruthenium as in Example 1, Step C, and reduced and tested as described in Example 1, Step D and E.

Example 8

A catalyst was prepared and tested as described in Example 1, but acid treatment (Example 1, Step B) was omitted and nickel impregnation (Example 1, Step A) was performed with nickel nitrate dissolved in the same amount of hydrochloric acid as given in Example 1, Step B, instead of with a saturated aqueous solution of nickel nitrate.

Example 9

A catalyst was prepared as described in Example 1, but the acid treatment (Example 1, Step B) was omitted and ruthenium impregnation (Example 1, Step C) was performed with ruthenium nitrosyl nitrate dissolved in the same amount of hydrochloric acid as given in Example 1, Step B, instead of with a two percent aqueous solution of ruthenium nitrosyl nitrate.

Example 10

A catalyst was prepared as described in Example 1, but acid treatment (Example 1, Step B) was omitted. After reduction at 400°C the catalyst was treated with hydrochloric acid as described in Example 1, Step B, then reduced again and tested as described in Example 1, Step D and E.

Example 11

A catalyst was prepared as described in Example 9, but ruthenium nitrosyl nitrate was replaced by the same molar amount of ruthenium chloride hydrate.

When testing the catalysts according to examples 7 to 11 the results given in the table were obtained.

Example 12

A catalyst was prepared and tested as described in Example 1, but a silica catalyst support containing 87% silicon dioxide and having a total surface area of 60 square meters per gram was used instead of alumina support. The results obtained are given in the table.

Example 13

A catalyst was prepared and tested as described in Example 1, but nickel nitrate was replaced by the same molar amount of cobaltous nitrate. The results obtained are given in the table.

Example 14

90 weight parts of catalyst support, consisting of 95 percent gamma alumina in form of tablets with length and diameter of 3 mm and a total surface area of about 100 square meters per gram support, were treated with hydrochloric acid as described in Example 1, Step B.

A saturated aqueous solution containing 10 weight parts of nickel and 0.5 weight parts of ruthenium, calculated as metals but applied as metal nitrates, was added to said hydrochloric acid treated catalyst support. Water was evaporated in vacuum at about 75°C, and the tablets were dried. This catalyst intermediate was then reduced and tested as described in Example 1, Step D and E. The results obtained are given in the table.

Example 15

A catalyst was prepared and tested as described in Example 1, but acid treatment (Step B) was omitted, and Nickel impregnation step (Step A) was modified by bubbling the air, used for decomposing of nickel nitrate, through an 18 percent aqueous solution of hydrogen chloride. The results obtained are given in the table.

Example 16

A catalyst was prepared and tested as described in Example 1, but acid treatment (Step B) was omitted, and Reduction with hydrogen (Step D) was performed with hydrogen gas bubbled through an 18 percent aqueous solution of hydrogen chloride. The results obtained are given in the table.

Example 17

89.5 weight parts of a catalyst support, consisting of 99 percent gamma alumina in form of tablets with length and diameter of 3 mm and a total surface area of about 80 square meters per gram support, were impregnated with an aqueous solution of nickel nitrate, ruthenium nitrosyl nitrate and hydrochloric acid. The solution contained 10 weight parts of nickel and 0.5 weight parts of ruthenium, both calculated as metals but applied as metal salts, and 1.1 weight parts of hydrogen chloride. Water was evaporated in vacuum at about 80°C, and the tablets were dried.

This catalyst intermediate was then reduced with hydrogen gas as described in Example 1, Step D, and tested as described in Example 1, Step E. The results obtained are reported in the table.

Table
=========

| Example no. | Conversion % | PRODUCTS weight percent | | | | | | AMINO GROUPS mole percent | | |
|---|---|---|---|---|---|---|---|---|---|---|
| | | EDA | PIP | DETA | AEP | AEEA | HEP | Primary | Secondary | Tertiary |
| 1 | 53.1 | 67.6 | 8.3 | 13.3 | 0.9 | 9.6 | 0.4 | 86 | 14 | 0.3 |
| 2 | 57.8 | 66.7 | 9.2 | 13.7 | 1.5 | 8.7 | 0.3 | 85 | 15 | 0.5 |
| 3 | 52.9 | 65.9 | 7.2 | 14.7 | 1.2 | 14.0 | 0.3 | 86 | 14 | 0.3 |
| 4 | 57.3 | 69.7 | 8.7 | 14.5 | 1.0 | 5.9 | 0.2 | 86 | 13 | 0.3 |
| 5 | 61.0 | 68.8 | 7.1 | 15.0 | 0.8 | 7.9 | 0.3 | 87 | 13 | 0.3 |
| 6 | 61.1 | 69.3 | 6.6 | 15.6 | 0.7 | 7.3 | 0.3 | 87 | 12 | 0.3 |
| Comp.A. | 59.0 | 64.5 | 14.5 | 14.2 | 1.8 | 4.6 | 0.6 | 82 | 18 | 0.6 |
| Comp.B. | 60.5 | 70.1 | 7.2 | 14.3 | 0.7 | 7.3 | 0.3 | 87 | 12 | 0.3 |
| 7 | 56.8 | 62.2 | 5.4 | 17.1 | 0.5 | 14.5 | 0.3 | 85 | 15 | 0.2 |
| 8 | 55.1 | 68.9 | 6.4 | 15.7 | 0.8 | 7.7 | 0.4 | 87 | 13 | 0.3 |
| 9 | 57.7 | 65.2 | 10.8 | 14.2 | 1.2 | 8.1 | 0.3 | 84 | 16 | 0.4 |
| 10 | 54.9 | 66.3 | 7.2 | 14.7 | 1.1 | 10.0 | 0.4 | 86 | 14 | 0.4 |
| 11 | 59.1 | 64.7 | 6.7 | 15.3 | 0.9 | 11.9 | 0.3 | 85 | 14 | 0.3 |
| 12 | 47.0 | 68.8 | 7.5 | 10.4 | 1.1 | 11.2 | 0.2 | 87 | 13 | 0.3 |
| 13 | 48.6 | 78.1 | 4.6 | 5.0 | 0.4 | 11.3 | 0.3 | 91 | 9 | 0.2 |
| 14 | 51.9 | 74.9 | 8.3 | 6.9 | 0.9 | 8.6 | 0.4 | 88 | 11 | 0.3 |
| 15 | 54.0 | 63.3 | 5.6 | 14.6 | 0.7 | 15.3 | 0.5 | 85 | 14 | 0.3 |
| 16 | 58.6 | 66.0 | 6.5 | 16.4 | 0.6 | 10.2 | 0.3 | 86 | 14 | 0.2 |
| 17 | 54.5 | 66.4 | 8.0 | 16.5 | 0.6 | 8.1 | 0.4 | 86 | 14 | 0.3 |

## Claims

1. A process for the preparation of a ruthenium-promoted halogen-containing, nickel and/or cobalt catalyst, containing from 4-40 % by weight, based on the total weight of said catalyst, of at least one metal selected from nickel and cobalt; from 0.1-5% by weight, based on the total weight of said catalyst, of ruthenium; and a porous metal oxide support comprised of at least 50% by weight of activated alumina, and/or silica, in which process the support is impregnated in one or more steps with a nickel compound

8

and/or a cobalt compound and a ruthenium compound, and the nickel and/or the cobalt compounds and the ruthenium compound being reduced into finely divided nickel and/or cobalt and ruthenium metals, characterized in that a halide is introduced into the catalyst by adding a halide compound in any other form than a ruthenium halide compound at any stage of the process.

2. A process as defined in claim 1, characterized in that the ruthenium compound is ruthenium nitrate or ruthenium nitrosyl nitrate.

3. A process as defined in claim 1 or 2, characterized in that the halide compound is hydrochloric acid.

4. A process as defined in any of claims 1 - 3, characterized in that the catalyst support is treated with the halide compound prior to applying nickel and/or cobalt onto it.

5. A process as defined in any of claims 1 - 4, characterized in that the catalyst support, coated with finely distributed nickel and/or cobalt as metal or oxide, is treated with the halide compound.

6. A process as defined in any of claims 1 - 4, characterized in that a catalyst intermediate, comprising support, nickel and/or cobalt as metal or oxide and ruthenium as metal, is treated with the halide compound.

7. A process as defined in any of claims 1 - 4, characterized in that the catalyst support is impregnated with a mixture of the nickel and/or cobalt compound, the ruthenium compound, and the halide compound prior to converting the nickel and/or cobalt and ruthenium compounds into finely divided metals.

**Revendications**

1. Procédé de préparation d'un catalyseur au nickel et/ou cobalt avec du ruthénium comme promoteur, contenant un halogène, ce catalyseur contenant de 4 à 40% en poids, calculé sur le poids total de ce catalyseur, d'au moins un métal choisi parmi le nickel et le cobalt; de 0,1 à 5% en poids, calculé sur le poids total de ce catalyseur, de ruthénium; et un support poreux en oxyde métallique comprenant au moins 50% en poids d'alumine activée et/ou silice, procédé d'après lequel on imprègne le support en une ou plusieurs étapes avec un composé de nickel et/ou un composé de cobalt et un composé de ruthénium, et on réduit les composés de nickel et/ou cobalt et le composé de ruthénium en nickel et/ou cobalt et en ruthénium sous forme de métaux finement divisés, ce procédé étant caractérisé par le fait qu'on introduit un halogénure dans le catalyseur en ajoutant à n'imprte quel stade du procédé un composé halogénure sous toute forme autre qu'un composé d'halogénure de ruthénium.

2. Procédé selon la revendication 1, caratérisé par le fait que le composé de ruthénium est du nitrate de ruthénium ou du nitrate de ruthénium nitrosyle.

3. Procédé selon l'une ou l'autre des revendications 1 ou 2, caractérisé par le fait que le composé halogénure est l'acide chlorydrique.

4. Procédé selon l'une quelconque des revendication 1 à 3, caractérisé par le fait que le support de catalyseur est traité par le composé halogénure avant qu'on y applique le nickel et/ou cobalt.

5. Procédé selon l'une quelconque des revendications 1 à 4, caractérisé par le fait que le support de catalyseur, recouvert de nickel et/ou cobalt finement distribué sous forme de métal ou d'oxyde, est traité par le composé halogénure.

6. Procédé selon l'une quelconque des revendications 1 à 4, caractérisé par le fait qu'un intermédiaire de catalyseur, comprenant le support, le nickel et/ou cobalt sous forme de métal ou d'oxyde et le ruthénium sous forme de métal, est traité par le composé halogénure.

7. Procédé selon l'une quelconque des revendications 1 à 4, caractérisé par le fait que le support de catalyseur est imprégné par un mélange du composé de nickel et/ou cobalt, du composé de ruthénium et du composé halogénure avant transformation des composés de nickel et/ou cobalt et de ruthénium en métaux finement divisés.

**Patentansprüche**

1. Verfahren zur Herstellung eines Ruthenium als Promotor enthaltenden, halogenhaltigen Nickel- und/oder Kobaltkatalysators, welcher enthält 4 bis 40 Gewichtsprozent - basierend auf dem Gesamtgewicht des Katalysators - von mindestens einem der Metalle Nickel und Kobalt; 0,1 bis 5 Gewichtsprozent - basierend auf dem Gesamtgewicht des Katalysators - Ruthenium; und einen porösen Metalloxidträger, umfassend mindestens 50 Gewichtsprozent an aktiviertem Aluminiumoxid und/oder Siliciumdioxid, in welchem Verfahren der Träger in einem oder mehreren Schritten mit einer Nickelverbindung und/oder einer Kobaltverbindung und einer Rutheniumverbindung imprägniert wird, die Nickel- und/oder Kobaltverbindung und die Rutheniumverbindung zu fein verteilten Nickel-und/oder Kobalt- und Rutheniummetallen reduziert werden, welches Verfahren dadurch gekennzeichnet ist, daß ein Halogenid in den Katalysator eingeführt wird durch Zugabe eines Halogenids beliebiger Form, außer der eines Rutheniumhalogenids, in einem beliebigen Stadium des Verfahrens.

2. Verfahren nach Anspruch 1, dadurch gekennzeichnet, daß die Rutheniumverbindung Rutheniumnitrat oder Rutheniumnitrosylnitrat ist.

3. Verfahren nach Anspruch 1 oder 2, dadurch gekennzeichnet, daß das Halogenid Salzsäure ist.

4. Verfahren nach irgendeinem der Ansprüche 1 bis 3,
   dadurch gekennzeichnet, daß der Katalysatorträger mit dem Halogenid behandelt wird, bevor Nickel und/oder Kobalt darauf aufgebracht werden.

5. Verfahren nach irgendeinem der Ansprüche 1 bis 4,
   dadurch gekennzeichnet, daß der Katalysatorträger, überzogen mit fein verteiltem Nickel und/oder Kobalt als Metall oder Metalloxid, mit dem Halogenid behandelt wird.

6. Verfahren nach irgendeinem der Ansprüche 1 bis 4,
   dadurch gekennzeichnet, daß ein Katalysatorzwischenprodukt, umfassend Träger, Nickel und/oder Kobalt als Metall oder Oxid und Ruthenium als Metall, mit dem Halogenid behandelt wird.

7. Verfahren nach irgendeinem der Ansprüche 1 bis 4,
   dadurch gekennzeichnet, daß der Katalysatorträger mit einer Mischung der Nickel- und/oder Kobaltverbindung, der Rutheniumverbindung und der Halogenidverbindung imprägniert wird, bevor die Nickel- und/oder Kobalt- und Rutheniumverbindungen in fein verteilte Metalle überführt werden.